# FASCICULE DE BREVET EUROPEEN

(11) **EP 2 332 910 B1**
(45) Date de publication et mention de la délivrance du brevet: **30.01.2013**
(21) Numéro de dépôt: 10290642.7
(22) Date de dépôt: 08.12.2010
(51) Int. Cl.: C07D 209/52, A61K 31/403, A61P 25/00

(54) **Nouveaux dérivés azabicyclo[3.2.0] hept-3-yl, leur procédé de préparation et les compositions pharmaceutiques qui les contiennent**
Neue Derivate von Azabicyclo[3.2.0]hept-3-yl, ihr Herstellungsverfahren und die pharmazeutischen Zusammensetzungen, die sie enthalten
New derivatives of azabicyclo[3.2.0]hept-3-yl, method for preparing same and pharmaceutical compositions containing them

(30) Priorité: 09.12.2009 FR 0905957
(43) Date de publication de la demande: 15.06.2011
(73) Titulaire: Les Laboratoires Servier, 92284 Suresnes Cedex (FR)
(72) Inventeur: Casara, Patrick, 78670 Villennes sur Seine (FR); Chollet, Anne-Marie, 78230 Le Pecq (FR); Dhainaut, Alain, 78400 Chatou (FR); Lestage, Pierre, 78170 La Celle Saint Cloud (FR); Panayi, Fany, 75018 Paris (FR)

(56) Documents cités:
- WO-A1-2005/089747

## Description

La présente invention concerne de nouveaux dérivés azabicyclo[3.2.0]hept-3-yl, leur procédé de préparation et les compositions pharmaceutiques qui les contiennent.

Les composés de la présente invention sont particulièrement intéressants d'un point de vue pharmacologique pour leur interaction avec les systèmes histaminergiques centraux *in vivo.*

Le vieillissement de la population, du fait de l'augmentation de l'espérance de vie à la naissance, a entraîné parallèlement un large accroissement de l'incidence des neuropathologies liées à l'âge, et notamment de la maladie d'Alzheimer. Les principales manifestations cliniques du vieillissement cérébral et surtout des neuropathologies liées à l'âge, sont les déficits des fonctions mnésiques et cognitives qui peuvent conduire à la démence.

Au niveau du système nerveux central, des études neuropharmacologiques ont montré que l'histamine *via* les systèmes histaminergiques centraux jouait un rôle de neurotransmetteur ou neuromodulateur en situations physiologiques ou physiopathologiques (Pell et Green, Annu. Rev. Neurosci., 1986, 9, 209-254; Schwartz et al., Physiol. Rev., 1991, 71, 1-51). Ainsi, il a été montré que l'histamine intervenait dans divers processus physiologiques et comportementaux tels que la thermorégulation, la régulation neuroendocrinienne, la nociception, le rythme circadien, les états cataleptiques, la motricité, l'agressivité, le comportement alimentaire, l'apprentissage et la mémorisation, ainsi que la plasticité synaptique (Hass et al., Histaminergic neurones : morphology and function, Boca Raton, FL : CRC Press, 1991, pp. 196-208 ; Brown et al., Prog. Neurobiology, 2001, 63, 637-672 ; Smith et al., Neuroimmunomodulation 2007, 14, pp. 317-325).

Des études, réalisées chez l'animal, ont montré que l'augmentation des taux endogènes extra-synaptiques d'histamine permettait de promouvoir les états de vigilance, les processus d'apprentissage et de mémoire et de réguler la prise alimentaire (Brown et al., Prog. Neurobiol., 2000, 63, 637-672; Passani et al., Neurosci. Biobehav. Rev., 2000, 24, 107-113). En conséquence, les indications thérapeutiques potentielles pour des composés capables d'augmenter le turn-over ou la libération d'histamine au niveau central sont le traitement des déficits cognitifs associés au vieillissement cérébral, aux maladies neurodégénératives aigues et chroniques, à la schizophrénie, ainsi que le traitement des troubles de l'humeur, du syndrome de Tourette (Gulhan Ercan-Sencicek et al, New England Journal of Medicine, May 20, 2010, 1901-1908), de la schizophrénie, des troubles du sommeil et du rythme veille-sommeil, du syndrome d'hyperactivité avec déficits attentionnels. Par ailleurs, des travaux ont montré qu'une injection d'histamine au niveau des noyaux centraux hypothalamiques impliqués dans la régulation de la satiété atténue l'alimentation chez le rat. De plus, un hypofonctionnement de la transmission histaminergique a été mis en évidence chez des rats génétiquement obèses (Machidori et al., Brain Research, 1992, 590, 180-186). En conséquence, les troubles du comportement alimentaire et l'obésité sont également des indications thérapeutiques potentielles pour les composés de la présente invention.

La présente invention concerne de nouveaux dérivés azabicycliques qui se distinguent des composés exemplifiés dans la demande WO2005/089747 par la présence d'un noyau 3-azabicyclo[3.2.0]heptane.

De manière surprenante, cette différence structurale par rapport aux composés de la demande WO2005/089747 confère aux composés de l'invention non seulement des propriétés procognitives remarquables, mais également de puissantes propriétés éveillantes, anti-sédatives, anti-hypnotiques et anxiolytiques.

Au niveau neurologique, cette combinaison d'activités ouvre la voie non seulement à de nouveaux traitements des troubles cognitifs liés au vieillissement cérébral, aux maladies neurodégénératives ou aux traumatismes crâniens, mais également au traitement des troubles psychocomportementaux associés à ces pathologies tels que les troubles du sommeil, l'apathie et/ou les états dépressifs. Par ailleurs, le profil pharmacologique des composés de l'invention permet aussi d'envisager de nouveaux traitements dans le domaine psychiatrique, pour le syndrome de Tourette, la schizophrénie, les troubles de l'humeur ou du sommeil par exemple.

La présente invention concerne plus particulièrement les composés de formule (I): 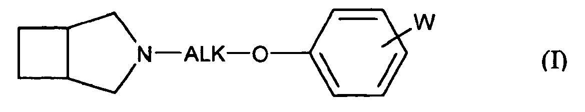 dans laquelle :
◆ ALK représente une chaîne alkylène,
◆ W représente un groupement 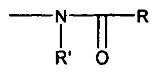 ou 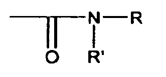
où R et R' représentent, indépendamment l'un de l'autre, un atome d'hydrogène ou un groupement alkyle (C₁-C₆) linéaire ou ramifié éventuellement substitué par un ou
plusieurs groupements choisis parmi halogène, hydroxy et alkoxy,
   étant entendu que :
   - le terme alkylène désigne un radical bivalent, linéaire ou ramifié, contenant de 2 à 6 atomes de carbone,
   - le terme alkoxy désigne un groupement alkyle-oxy dont la chaîne alkyle, linéaire ou ramifiée, contient de 1 à 6 atomes de carbone,
   leurs énantiomères et diastéréoisomères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléique, citrique, ascorbique, oxalique, méthane sulfonique, camphorique, etc.

Parmi les bases pharmaceutiquement acceptables, on peut citer à titre non limitatif l'hydroxyde de sodium, l'hydroxyde de potassium, la triéthylamine, la tertbutylamine, etc. Les composés de formule (I) préférés sont ceux pour lesquels le groupement W est situé en position para.

De manière préférentielle, ALK représente un radical bivalent linéaire contenant de 2 à 6 atomes de carbone comme, par exemple, le groupe éthylène, propylène ou butylène, et plus préférentiellement encore un groupe propylène.

Un aspect particulier de l'invention concerne les composés de formule (I) pour lesquels W représente le groupement

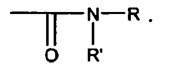

Un autre aspect particulier de l'invention concerne les composés de formule (I) pour lesquels W représente le groupement

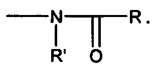

De manière avantageuse, R et R' représentent, indépendamment l'un de l'autre, un atome d'hydrogène, un groupement méthyle ou un groupement éthyle, ces groupements étant éventuellement substitués par un méthoxy.

Plus particulièrement, W représente le groupement -CO-NH-CH₃, -CO-N(CH₃)₂, -CO-NH₂, -CO-N(CH₂CH₃)₂, -NH-CO-CH₃, -N(CH₃)-CO-CH₃ ou -NH-CO-CH₂-OCH₃.

Les composés de configuration *meso* sont plus particulièrement préférés.

Encore plus particulièrement, l'invention concerne les composés de formule (I) qui sont :
- le 4-{3-[(1*R*,5*S*)-3-azabicyclo[3.2.0]hept-3-yl]propoxy}benzamide,
- le *N*-(4-{3-[(1*R*,5*S*)-3-azabicyclo[3.2.0]hept-3-yl]propoxy}phényl)acétamide,
- le 4-{3-[(1*R*,5*S*)-3-azabicyclo[3.2.0]hept-3-yl]propoxy}-*N*,*N*-diméthylbenzamide,
- le *N*-(4-{3-[(1*R*,5*S*)-3-azabicyclo[3.2.0]hept-3-yl]propoxy}phényl)-*N*-méthyl acétamide,
et leurs sels d'addition à un acide ou à une base pharmaceutiquement acceptables.

Parmi les sels d'addition à un acide pharmaceutiquement acceptable, on préfère plus particulièrement les chlorhydrate, oxalate et citrate.

L'invention s'étend également au procédé de préparation des composés de formule (I) **caractérisé en ce que** l'on utilise comme produit de départ le composé de formule (II) : 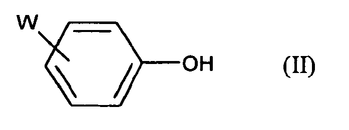 dans laquelle W est tel que défini dans la formule (I),
composé de formule (II) sur lequel on condense en milieu basique le composé de formule (III) : 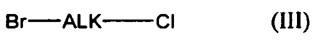 dans laquelle ALK est tel que défini dans la formule (I),
pour obtenir le composé de formule (IV) : 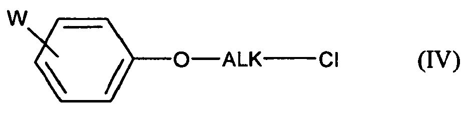 dans laquelle W et ALK sont tels que définis précédemment, sur lequel on condense le composé de formule (V) : 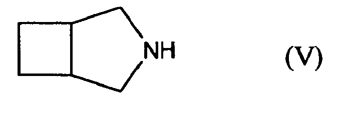 pour conduire au composé de formule (I) tel que défini précédemment : 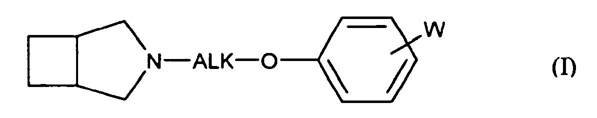 qui peut être purifié selon une technique classique de séparation, que l'on transforme, si on le souhaite, en ses sels d'addition à un acide ou à une base pharmaceutiquement acceptable et dont on sépare éventuellement les isomères optiques selon une technique classique de séparation.

Les composés de formules (II), (III) et (V) sont soit commerciaux, soit accessibles à l'homme du métier par des réactions chimiques classiques et décrites dans la littérature.

Alternativement, les composés de formule (VI) : 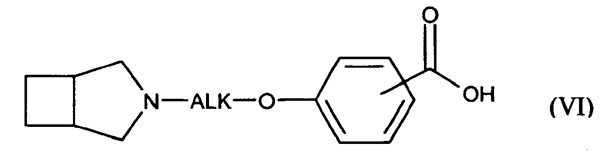 dans lesquels le groupement ALK est tel que défini précédemment,
peuvent être utilisés en tant qu'intermédiaires de synthèse des composés de formule (I/a), cas particuliers des composés de formule (I) pour lesquels W représente un groupement -CONRR', par couplage avec une amine de formule NHRR', où R et R' ont la même signification que dans la formule (I).

De même, les composés de formule (VII) : 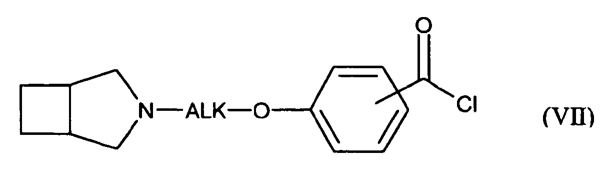 dans lesquels le groupement ALK est tel que défini précédemment,
peuvent être utilisés en tant qu'intermédiaires de synthèse des composés de formule (I/a), cas particuliers des composés de formule (I) pour lesquels W représente un groupement -CONRR', par couplage avec une amine de formule NHRR', où R et R' ont la même signification que dans la formule (I).

De plus, les composés de formule (I/a), cas particuliers des composés de formule (I) pour lesquels W représente un groupement -CONRR', peuvent aussi être obtenus par condensation de l'amine NHRR', où R et R' ont la même signification que dans la formule (I), en utilisant les composés de formule (VIII) : 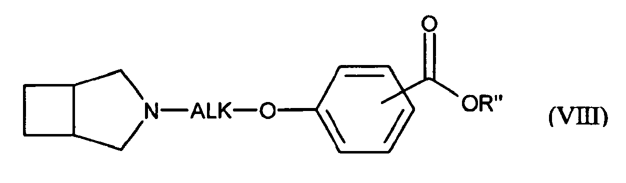 dans lesquels le groupement ALK est tel que défini précédemment et R" représente un groupement alkyle (C₁-C₆) linéaire ou ramifié ou un groupement benzyle,
les composés de formule (VIII) étant préparés *via* l'acide carboxylique (VI) ou le chlorure d'acyle (VII) correspondant présentés précédemment.

Enfin, il est également possible d'obtenir les composés de formule (I/a) en hydrolysant des composés de formule (IX) : 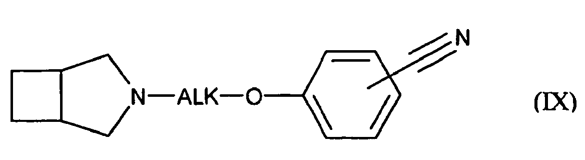 dans lesquels le groupement ALK est tel que défini précédemment. L'étude pharmacologique des composés de formule (I) a montré qu'ils possédaient des propriétés procognitives en facilitant les processus de mémorisation et d'apprentissage, des propriétés éveillantes, anti-sédatives, anti-hypnotiques et anxiolytiques.

Au niveau neurologique, les composés selon l'invention peuvent être utiles dans le traitement des troubles cognitifs liés au vieillissement cérébral ou aux maladies neurodégénératives telles que la maladie d'Alzheimer, la maladie de Parkinson, la maladie de Pick, les démences à corps de Lewy, les démences frontales et sous-corticales, les démences frontotemporales, les démences vasculaires, la maladie de Huntington, la sclérose en plaques, dans de nouveaux traitements des troubles cognitifs liés aux traumatismes crâniens, mais également dans le traitement des troubles psychocomportementaux associés à ces pathologies tels que les troubles du sommeil, l'apathie et les états anxio-dépressifs. Les troubles du sommeil associés à la maladie d'Alzheimer et à la maladie de Parkinson, tels que les hypersomnolences diurnes, sont particulièrement visés. Par ailleurs, les troubles moteurs associés à la maladie de Parkinson peuvent également être traités par les composés de la présente invention.

Au niveau psychiatrique, ces composés peuvent être utiles dans le traitement des troubles de l'humeur, et plus particulièrement le traitement des états anxio-dépressifs, du syndrome de Tourette, de la schizophrénie et des troubles cognitifs qui lui sont associés, de la douleur, ainsi que dans le traitement des troubles du sommeil, du rythme veille-sommeil et du syndrome d'hyperactivité avec déficits attentionnels (ADHD). Parmi les troubles du sommeil, on peut citer plus particulièrement la narcolepsie et l'apnée du sommeil. Les troubles du sommeil tels que les hypersomnies survenant lors du syndrome d'apnée obstructive du sommeil ou du syndrome d'hyperactivité avec déficits attentionnels, ainsi que les somnolences diurnes sont également visés.

La présente invention a également pour objet les compositions pharmaceutiques contenant un composé de formule (I) en combinaison avec un ou plusieurs excipients pharmaceutiquement acceptables.

Dans les compositions pharmaceutiques selon l'invention, la fraction massique en principe actif (masse du principe actif sur la masse totale de la composition) est comprise entre 1 et 50%.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,05 mg et 500 mg par 24 heures pour un traitement en 1 à 3 prises par jour.

L'association d'un composé de formule (I) avec la L-dopa fait aussi partie intégrante de l'invention, et plus particulièrement encore l'association du 4-{3-[(1*R*,5*S*)-3-azabicyclo[3.2.0]hept-3-yl]propoxy}benzamide, ou d'un de ses sels d'addition à un acide ou à une base pharmaceutiquement acceptable, avec la L-dopa. Les associations de ce type peuvent être utilisées dans le traitement des troubles cognitifs et moteurs de la maladie de Parkinson.

Les exemples suivants illustrent l'invention et ne la limitent en aucune façon. Les structures des composés, décrits dans les exemples, ont été déterminées selon les techniques spectrophotométriques usuelles (infrarouge, RMN, spectrométrie de masse...).

A titre d'information, tous les composés ci-dessous présentent une stéréochimie de type *meso.*

### Exemple 1, voie de synthèse A : Chlorhydrate de 4-{3-[(1R,5S)-3-azabicyclo[3.2.0] hept-3-yl]propoxy}benzamide

### Stade 1 : 4-(3-Chloropropoxy)benzamide

Un mélange constitué de 0,004 mole de 4-hydroxybenzamide, de 0,004 mole de 1-bromo-3-chloropropane et de 0,006 mole de carbonate de césium dans 10ml d'acétonitrile est chauffé au reflux pendant 5 heures.

### Stade 2 : 4-{3-[(1R,5S)-3-Azabicyclo[3.2.0]hept-3-yl]propoxy}benzamide

Dans le milieu réactionnel du Stade 1 à température ambiante sont ajoutées 0,004 mole de (1*R*,5*S*)-3-azabicyclo[3.2.0]heptane, dont la synthèse est décrite dans la publication J. Med. Chem. 1967, 10, 621-623, et 0,002 mole d'iodure de sodium. Le chauffage au reflux est ensuite repris pendant 16 heures. Le précipité est filtré, rincé avec de l'acétonitrile. Le filtrat est concentré à sec. Le résidu est repris dans du dichlorométhane. Cette solution est extraite avec de la soude, puis de l'eau, avant d'être séchée sur sulfate de magnésium et concentrée à sec. Le résidu est purifié par technique de chromatographie préparative sur phase Lichroprep RP-18.

### Spectre de masse : [M+H]⁺ m/z théorique = 275,1760 ; m/z expérimental = 275,1773

### Stade 3 : Chlorhydrate de 4-{3-[(1R,5S)-3-azabicyclo[3.2.0]hept-3-yl]propoxy}benzamide

Le produit obtenu au Stade 2 est dissous dans 10 ml d'éthanol auxquels sont ajoutés 2 ml d'éther chlorhydrique 2 N. Le produit ainsi obtenu est filtré, rincé avec de l'éthanol et séché sous vide.

| *Microanalyse élémentaire :* | | | | |
|---|---|---|---|---|
| | % *C* | *% H* | *% N* | *%Cl-* |
| *Calculé* | *61, 83* | *7, 46* | *9, 01* | *11, 41* |
| *Trouvé* | *61,33* | *7,37* | *8,85* | *11,50* |

### Exemple 1, voie B: Chlorhydrate de 4-{3-[(1R,5S)-3-azabicyclo[3.2.0]hept-3-yl] propoxy}benzamide

### Stade 1 : 4-{3-[(1R,5S)-3-Azabicyclo[3.2.0]hept-3-yl]propoxy}benzonitrile

Le procédé expérimental est identique aux Stades 1 et 2 de l'Exemple 1, voie de synthèse A, en remplaçant au Stade 1 le 4-hydroxybenzamide par le 4-hydroxybenzonitrile.

### Stade 2 : Chlorhydrate de 4-{3-[(1R,5S)-3-azabicyclo[3.2.0]hept-3-yl]propoxy}benzamide

Le composé obtenu au stade précédent (2,2 g) est mis en solution dans 90 ml d'éthanol et chauffé à reflux en présence de 5,1 g de KOH pendant 18 heures. Le milieu est versé dans 90 ml d'eau puis concentré à mi-volume sous vide. Le solide obtenu est filtré, rincé avec de l'éther isopropylique, puis séché. Le chlorhydrate est préparé suivant le mode opératoire du Stade 3 de l'Exemple 1, voie de synthèse A.

| *Microanalyse élémentaire :* | | | | |
|---|---|---|---|---|
| | *% C* | *%H* | *%N* | *%Cl-* |
| *Calculé* | *61,83* | *7,46* | *9,01* | *11,41* |
| *Trouvé* | *61, 69* | *7, 39* | *8, 77* | *11, 47* |

### Exemple 1, voie C : Chlorhydrate de 4-{3-[(1R,5S)-3-azabicyclo[3.2.0]hept-3-yl] propoxy}benzamide

### Stade 1 : 4-{3-[(1R,5S)-3-Azabicyclo[3.2.0]hept-3-yl]propoxy}benzoate de méthyle

Le procédé expérimental est identique aux Stades 1 et 2 de l'Exemple 1, voie de synthèse A, en remplaçant au Stade 1 le 4-hydroxybenzamide par le 4-hydroxybenzoate de méthyle.

### Stade 2 : Acide 4-{3-[(1R,5S)-3-azabicyclo[3.2.0]hept-3-yl]propoxy}benzoïque

Un mélange de 3,5 g du composé du Stade 1, de 12,7 ml de soude 2 N et de 8 ml de méthanol est chauffé à reflux pendant une heure. Dans le milieu réactionnel refroidi au bain de glace sont ajoutés 12,7 ml d'HCl 2 N. Le précipité est lavé à l'eau et séché sous vide.

| *Microanalyse élémentaire :* | | | |
|---|---|---|---|
| | *%C* | *%H* | *%N* |
| *Calculé* | *69, 79* | *7, 69* | *5, 09* |
| *Trouvé* | *69, 67* | *7, 73* | *5, 44* |

### Stade 3 : Chlorure de 4-{3-[(1R,5S)-3-azabicyclo[3.2.0]hept-3-yl]propoxy}benzoyle, chlorhydrate

Un mélange de 1,8 g de produit décrit au Stade 2 et de 20 ml de chlorure de thionyle est chauffé à reflux pendant 2 heures. Le milieu réactionnel est concentré sous vide, coévaporé deux fois avec du toluène. Le résidu solide est homogénéisé dans l'éther éthylique, filtré et séché sous vide.

### Stade 4 : Chlorhydrate de 4-{3-[(1R,5S)-3-azabicyclo[3.2.0]hept-3-yl]propoxy}benzamide

Dans une solution de 1 g de produit décrit au Stade 3 dans le dichlorométhane à 0 °C, sont ajoutés goutte à goutte 4 ml de méthanol ammoniacal 2 N. Le mélange est ensuite agité 1 heure à température ambiante, lavé avec un solution de soude 2 N, puis de l'eau. La phase organique est séchée sur sulfate de magnésium et concentrée. L'huile résiduelle est dissoute dans 10 ml d'éthanol auxquels sont ajoutés 2 ml d'éther chlorhydrique 2 N. Le produit ainsi obtenu est filtré, rincé avec de l'éthanol et séché sous vide.

| *Microanalyse élémentaire :* | | | | |
|---|---|---|---|---|
| | *%C* | *%H* | *%N* | *%Cl-* |
| *Calculé* | *61,83* | *7,46* | *9,01* | *11,41* |
| *Trouvé* | *61,57* | *7,40* | *8,71* | *11,53* |

### Exemple 2 : 4-{3-[(1R,5S)-3-Azabicyclo[3.2.0]hept-3-yl]propoxy}benzamide

Le procédé expérimental reprend les Stades 1 et 2 de l'Exemple 1, voie de synthèse A.

| *Microanalyse élémentaire :* | | | |
|---|---|---|---|
| | *%C* | *%H* | *%N* |
| *Calculé* | *70,04* | *8,08* | *10,21* |
| *Trouvé* | *69,24* | *7,58* | *9,76* |

### Exemple 3 : 4-{2-[(1R,5S)-3-Azabicyclo[3.2.0]hept-3-yl]éthoxy}benzamide

Le procédé expérimental est identique aux Stades 1 et 2 de l'Exemple 1, voie de synthèse A, en remplaçant au Stade 1 le 1-bromo-3-chloropropane par le 1-bromo-2-chloroéthane.

| *Microanalyse élémentaire :* | | | |
|---|---|---|---|
| | *%C* | *%H* | *%N* |
| *Calculé* | *69,21* | *7,74* | *10,76* |
| *Trouvé* | *69,00* | *7,72* | *10,58* |

### Exemple 4 : 4-{4-[(1R,5S)-3-Azabicyclo[3.2.0]hept-3-yl]butoxy}benzamide

Le procédé expérimental est identique aux Stades 1 et 2 de l'Exemple 1, voie de synthèse A, en remplaçant au Stade 1 le 1-bromo-3-chloropropane par le 1-bromo-4-chlorobutane.

| *Microanalyse élémentaire :* | | | |
|---|---|---|---|
| | *%C* | *%H* | *%N* |
| *Calculé* | *70,80* | *8,39* | *9,71* |
| *Trouvé* | *69,18* | *8,28* | *9,37* |

### Exemple 5 : N-(4-{3-[(1R,5S)-3-Azabicyclo[3.2.0]hept-3-yl]propoxy}phényl)acétamide

Le procédé expérimental est identique aux Stades 1 et 2 de l'Exemple 1, voie de synthèse A, en remplaçant au Stade 1 le 4-hydroxybenzamide par le *N*-(4-hydroxyphényl) acétamide.

| *Microanalyse élémentaire :* | | | |
|---|---|---|---|
| | *%C* | *%H* | *%N* |
| *Calculé* | *70,80* | *8,39* | *9,71* |
| *Trouvé* | *70,54* | *8,35* | *10,22* |

### Exemple 6 : N-(4-{2-[(1R,5S)-3-Azabicyclo[3.2.0]hept-3-yl]éthoxy}phényl)acétamide

Le procédé expérimental est identique à l'Exemple 3 en remplaçant le 4-hydroxybenzamide par le *N*-(4-hydroxyphényl)acétamide.

| *Microanalyse élémentaire :* | | | |
|---|---|---|---|
| | *%C* | *%H* | *%N* |
| *Calculé* | *70,04* | *8,08* | *10,21* |
| *Trouvé* | *69,44* | *7,96* | *9,96* |

### Exemple 7 : N-(4-{4-[(1R,5S)-3-Azabicyclo[3.2.0]hept-3-yl]butoxy}phényl)acétamide

Le procédé expérimental est identique à l'Exemple 4 en remplaçant le 4-hydroxybenzamide par le *N*-(4-hydroxyphényl)acétamide.

| *Microanalyse élémentaire :* | | | |
|---|---|---|---|
| | *%C* | *%H* | *%N* |
| *Calculé* | *71,49* | *8, 67* | *9, 26* |
| *Trouvé* | *71,02* | *8,58* | *8,93* |

### Exemple 8: Chlorhydrate de 4-{3-[(1R,5S)-3-azabicyclo[3.2.0]hept-3-yl]propoxy}-N,N-diméthylbenzamide

Le procédé expérimental est identique à celui de l'Exemple 1, voie de synthèse A, en remplaçant au Stade 1 le 4-hydroxybenzamide par le 4-hydroxy-*N*,*N*-diméthylbenzamide.

| *Microanalyse élémentaire :* | | | | |
|---|---|---|---|---|
| | *%C* | *%H* | *%N* | *%Cl-* |
| *Calculé* | *63,80* | *8,03* | *8,27* | *10,46* |
| *Trouvé* | *63,29* | *7,95* | *8,19* | *10,50* |

### Exemple 9 : Chlorhydrate de 4-{2-[(1R,5S)-3-azabicyclo[3.2.0]hept-2-yl]éthoxy}-N,N-diméthylbenzamide

Le procédé expérimental est identique à l'Exemple 3 en remplaçant le 4-hydroxybenzamide par le 4-hydroxy-*N*,*N*-diméthylbenzamide. Le composé ainsi obtenu est salifié suivant le mode opératoire du Stade 3 de l'Exemple 1, voie de synthèse A.

| *Microanalyse élémentaire :* | | | | | |
|---|---|---|---|---|---|
| | *%C* | *%H* | *%N* | *%Cl* | *%Cl-* |
| *Calculé* | *62,86* | *7,76* | *8, 62* | *10,91* | *10,91* |
| *Trouvé* | *62,54* | *7, 66* | *8,40* | *10,66* | *10,67* |

### Exemple 10 : Chlorhydrate de 4-{4-[(1R,5S)-3-azabicyclo[3.2.0]hept-3-yl]butoxy}-N,N-diméthylbenzamide

Le procédé expérimental est identique à l'Exemple 4 en remplaçant le 4-hydroxybenzamide par le 4-hydroxy-*N*,*N*-diméthylbenzamide. Puis, on ajoute 2 ml d'acide chlorhydrique 2 N au composé ainsi obtenu dans 10 ml d'éthanol. Le produit obtenu est filtré, rincé à l'éther éthylique puis séché sous vide.

| *Microanalyse élémentaire :* | | | | |
|---|---|---|---|---|
| | *%C* | *%H* | *%N* | *%Cl-* |
| *Calculé* | *64,67* | *8, 28* | *7,94* | *10,05* |
| *Trouvé* | *64,39* | *7,76* | *7,94* | *10,62* |

### Exemple 11 : Chlorhydrate de 4-{3-[(1R,5S)-3-azabicyclo[3.2.0]hept-3-yl]propoxy}-N,N-diéthylbenzamide

Le procédé expérimental est identique à celui de l'Exemple 1, voie de synthèse A, en remplaçant au Stade 1 le 4-hydroxybenzamide par le 4-hydroxy-*N*,*N*-diéthylbenzamide.

| *Microanalyse élémentaire :* | | | | | |
|---|---|---|---|---|---|
| | *%C* | *%H* | *%N* | *%Cl* | *%Cl-* |
| *Calculé* | *65,47* | *8,52* | *7,63* | *9,66* | *9,66* |
| *Trouvé* | *64,66* | *8, 25* | *7,59* | *10,56* | *10,13* |

### Exemple 12 : Chlorhydrate de 4-{2-[(1R,5S)-3-azabicyclo[3.2.0]hept-3-yl]éthoxy}-N,N-diéthylbenzamide

Le procédé expérimental est identique à l'Exemple 3 en remplaçant le 4-hydroxybenzamide par le 4-hydroxy-*N*,*N*-diéthylbenzamide. Le composé ainsi obtenu est salifié suivant le mode opératoire du Stade 3 de l'Exemple 1, voie de synthèse A.

| *Microanalyse élémentaire :* | | | | |
|---|---|---|---|---|
| | *%C* | *%H* | *%N* | *%Cl-* |
| *Calculé* | *64,67* | *8, 28* | *7,94* | *10,05* |
| *Trouvé* | *64,57* | *7,91* | *8, 03* | *9, 65* |

### Exemple 13 : Chlorhydrate de 4-{4-[(1R,5S)-3-Azabicyclo[3.2.0]hept-3-yl]butoxy}-N,N-diéthylbenzamide

Le procédé expérimental est identique à l'Exemple 4 en remplaçant le 4-hydroxybenzamide par le 4-hydroxy-*N*,*N*-diéthylbenzamide. Puis, on ajoute 2 ml d'acide chlorhydrique 2 N au composé ainsi obtenu dans 10 ml d'éthanol. Le produit obtenu est filtré, rincé à l'éther éthylique puis séché sous vide

| *Microanalyse élémentaire :* | | | | |
|---|---|---|---|---|
| | *%C* | *%H* | *%N* | *%Cl-* |
| *Calculé* | *66,21* | *8,73* | *7,35* | *9,31* |
| *Trouvé* | *67,41* | *8,34* | *7,67* | *12,01* |

### Exemple 14 : Chlorhydrate de 4-{3-[(1R,5S)-3-azabicyclo[3.2.0]hept-3-yl]propoxy}-N-méthylbenzamide

Le procédé expérimental est identique à celui de l'Exemple 1, voie de synthèse A, en remplaçant au Stade 1 le 4-hydroxybenzamide par le 4-hydroxy-*N*-méthylbenzamide.

| *Microanalyse élémentaire :* | | | | | |
|---|---|---|---|---|---|
| | *%C* | *%H* | *%N* | *%Cl* | *%Cl-* |
| *Calculé* | *62,86* | *7,76* | *8,62* | *10,91* | *10,91* |
| *Trouvé* | *62,57* | *7,68* | *8,61* | *11,10* | *10,99* |

### Exemple 15 : 4-{2-[(1R,5S)-3-Azabicyclo[3.2.0]hept-2-yl]éthoxy}-N-méthylbenzamide

Le procédé expérimental est identique à l'Exemple 3 en remplaçant le 4-hydroxybenzamide par le 4-hydroxy-*N*-méthylbenzamide.

| *Microanalyse élémentaire :* | | | |
|---|---|---|---|
| | *%C* | *%H* | *%N* |
| *Calculé* | *70,04* | *8, 08* | *10,21* |
| *Trouvé* | *69,66* | *7,98* | *10,12* |

### Exemple 16 : 4-{4-[(1R,5S)-3-Azabicyclo[3.2.0]hept-3-yl]butoxy}-N-méthylbenzamide

Le procédé expérimental est identique à de l'Exemple 4 en remplaçant le 4-hydroxybenzamide par le 4-hydroxy-*N*-méthylbenzamide.

**Microanalyse élémentaire :**

| | *%C* | *%H* | *%N* |
|---|---|---|---|
| *Calculé* | *71,49* | *8, 67* | *9, 26* |
| *Trouvé* | *70,99* | *8,47* | *8,40* |

### Exemple 17: Chlorhydrate de N-(4-{3-[(1R,5S)-3-azabicyclo[3.2.0]hept-3-yl] propoxy}phényl)-N-méthylacétamide

Le procédé expérimental est identique à celui de l'Exemple 1, voie de synthèse A, en remplaçant au Stade 1 le 4-hydroxybenzamide par le *N*-(4-hydroxyphényl)-*N-*méthylacétamide.

| *Microanalyse élémentaire.* | | | | | |
|---|---|---|---|---|---|
| | *%C* | *%H* | *%N* | *% Cl* | *%Cl-* |
| *Calculé* | *63,80* | *8, 03* | *8,27* | *10,46* | *10,46* |
| *Trouvé* | *64,07* | *7,97* | *7,88* | *10,16* | *10,30* |

### Exemple 18: Chlorhydrate de N-(4-{2-[(1R,5S)-3-azabicyclo[3.2.0]hept-2-yl]éthoxy} phényl)-N-méthylacétamide

Le procédé expérimental est identique à l'Exemple 3 en remplaçant le 4-hydroxybenzamide par le *N*-(4-hydroxyphényl)-*N*-méthylacétamide. Le composé ainsi obtenu est salifié suivant le mode opératoire du Stade 3 de l'Exemple 1, voie de synthèse A.

| *Microanalyse élémentaire :* | | | | | |
|---|---|---|---|---|---|
| | *% C* | *%H* | *%N* | *% Cl* | *% Cl⁻* |
| *Calculé* | *62,86* | *7,76* | *8,62* | *10,91* | *10,91* |
| *Trouvé* | *62,14* | *7,86* | *8, 06* | *11,32* | *10,73* |

### Exemple 19: Chlorhydrate de N-(4-{4-[(1R,5S)-3-azabicyclo[3.2.0]hept-3-yl]butoxy} phényl)-N-méthylacétamide

Le procédé expérimental est identique à l'Exemple 13 en remplaçant le 4-hydroxy-*N*,*N* diéthylbenzamide par le *N*-(4-hydroxyphényl)-*N*-méthylacétamide.

| *Microanalyse élémentaire :* | | | | | |
|---|---|---|---|---|---|
| | *%C* | *%H* | *%N* | *%Cl* | *% Cl⁻* |
| *Calculé* | *64,67* | *8, 28* | *7,94* | *10,05* | *10,05* |
| *Trouvé* | *64,25* | *7,79* | *7,89* | *10,47* | *10,14* |

### Exemple 20 : N-(4-{3-[(1R,5S)-3-Azabicyclo[3.2.0]hept-3-yl]propoxy}phényl)-2-méthoxyacétamide

Le procédé expérimental est identique aux stades 1 et 2 de l'Exemple 1, voie de synthèse A, en remplaçant au Stade 1 le 4-hydroxybenzamide par le *N*-(4-hydroxyphényl)-2-méthoxyacétamide.

| *Microanalyse élémentaire.* | | | |
|---|---|---|---|
| | *%C* | *%H* | *%N* |
| *Calculé* | *67,90* | *8, 23* | *8,80* |
| *Trouvé* | *67,88* | *8, 22* | *8,97* |

### Exemple 21: Chlorhydrate de N-(4-{2-[(1R,5S)-3-azabicyclo[3.2.0]hept-3-yl]éthoxy} phényl)-2-méthoxyacétamide

Le procédé expérimental est identique à l'Exemple 3 en remplaçant le 4-hydroxybenzamide par le *N*-(4-hydroxyphényl)-2-méthoxyacétamide. Puis, on ajoute 2 ml d'acide chlorhydrique 2 N au composé ainsi obtenu dans 10 ml d'éthanol. Le produit obtenu est filtré, rincé à l'éther éthylique puis séché sous vide.

| *Microanalyse élémentaire :* | | | | | |
|---|---|---|---|---|---|
| | *%C* | *%H* | *%N* | *%Cl* | *% Cl⁻* |
| *Calculé* | *59,91* | *7,39* | *8,22* | *10,40* | *10,40* |
| *Trouvé* | *59,76* | *7,44* | *8,12* | *10,66* | *10,36* |

### Exemple 22: Chlorhydrate de N-(4-{4-[(1R,5S)-3-azabicyclo[3.2.0]hept-3-yl]butoxy} phényl)-2-méthoxyacétamide

Le procédé expérimental est identique à l'Exemple 13 en remplaçant le 4-hydroxy-*N*,*N* diéthylbenzamide par le *N*-(4-hydroxyphényl)-2-méthoxyacétamide.

| *Microanalyse élémentaire.* | | | | | |
|---|---|---|---|---|---|
| | *%C* | *%H* | *%N* | *% Cl* | *% Cl⁻* |
| *Calculé* | *61,86* | *7,92* | *7,59* | *9,61* | *9,61* |
| *Trouvé* | *61,61* | *7,83* | *7,42* | *9,80* | *9,41* |

### Exemple 23 : Oxalate de 4-{3-[(1R,5S)-3-azabicyclo[3.2.0]hept-3-yl]propoxy} benzamide

Le procédé expérimental reprend les Stades 1 et 2 de l'Exemple 1, voie de synthèse A. Puis, on ajoute 0,32 g d'acide oxalique à 0,38 g du composé ainsi obtenu dans 6 ml d'éthanol. Le produit obtenu est filtré, rincé à l'éther éthylique puis séché sous vide.

| *Microanalyse élémentaire.* | | | |
|---|---|---|---|
| | *%C* | *%H* | *%N* |
| *Calculé* | *59,33* | *6,64* | *7,69* |
| *Trouvé* | *58,99* | *6,61* | *7,49* |

### Exemple 24: Citrate de 4-{3-[(1R,5S)-3-azabicyclo[3.2.0]hept-3-yl]propoxy} benzamide

On dissout un équivalent du composé de l'Exemple 1, voie de synthèse A, Stade 2 en présence de 1,2 équivalent d'acide citrique monohydraté dans l'eau pour conduire au produit du titre.

| *Microanalyse élémentaire :* | | | |
|---|---|---|---|
| | *%C* | *%H* | *%N* |
| *Calculé* | *56,65* | *6,48* | *6,01* |
| *Trouvé* | *56,49* | *6,60* | *5,99* |

### ETUDE PHARMACOLOGIQUE

### EXEMPLE A : Dosages cérébraux de la N^{τ}-Méthylhistamine chez la souris NMRI

Cette étude réalisée selon la méthode de Taylor et Coll. (Biochem. Pharm., 1992, 44, 1261-1267), a pour objectif d'évaluer l'activité *ex vivo* des composés de la présente invention en tant qu'antagonistes des récepteurs histaminergiques centraux de type H₃. Cette activité est révélée par la mesure, après traitement par voie orale des composés sous étude, des taux centraux de N^{τ}-Méthylhistamine, métabolite principal de l'histamine. Une augmentation des concentrations cérébrales de N^{τ}-Méthylhistamine signe une augmentation du turn-over de l'histamine par blocage des récepteurs histaminergiques centraux de type H₃.

Des souris NMRI (18-20 g) sont traitées par voie orale par les composés de la présente invention ou par leur véhicule (20 ml/kg). Une heure après le traitement pharmacologique, les animaux sont sacrifiés, les cerveaux sont prélevés, congelés dans l'azote liquide, pesés et homogénéisés dans HClO₄ 0,1 N à 4 °C. Les homogénats sont centrifugés (15000 g, 17 minutes, 4 °C). Les surnageants sont récupérés et aliquotés. Les aliquotes sont congelés dans l'azote liquide et stockés à -80 °C jusqu'à leur analyse.
La détermination des taux cérébraux de N^{τ}-Méthylhistamine est réalisée par électrophorèse capillaire. Les taux tissulaires de N^{τ}-Méthylhistamine sont exprimés en µg/g de cerveau frais. La comparaison des taux cérébraux de N^{τ}-Méthylhistamine entre les animaux traités par le véhicule (témoins) et les animaux traités par les composés de la présente invention est effectuée par une analyse de variance à un facteur suivie si nécessaire par une analyse complémentaire (test de Dunnett).

Les résultats montrent que les composés de la présente invention sont capables, à la dose de 3 mg/kg PO, d'augmenter significativement les concentrations cérébrales endogènes de N^{τ}-Méthylhistamine. Ainsi, les composés des Exemples 1, 5, 8 et 17 augmentent de plus de 100 % les concentrations cérébrales de N^{τ}-Méthylhistamine

### EXEMPLE B : Enregistrements de l'électroencéphalogramme chez le rat Wistar vigile

Des rats Wistar mâles adultes sont implantés de manière chronique par des électrodes placées à la surface du cortex frontal et pariétal. Des enregistrements de l'électroencéphalogramme (EEG) cortical sont effectués chez les rats placés dans des cages dans une pièce isolée phoniquement. Les composés et véhicules sont administrés par voie intrapéritonéale de manière randomisée à 10 heures les mêmes jours avec un minimum de 3 jours entre chaque administration, permettant d'utiliser chaque rat comme son propre témoin. La puissance absolue des activité lentes delta (1-4 Hz), qui prédominent pendant le sommeil lent et disparaissent pendant l'éveil et le sommeil paradoxal, est moyennée sur des périodes successives de 30 minutes. Sur 30 minutes, des valeurs élevées et basses de la puissance des activités lentes delta sont des signes d'éveil et de sommeil, respectivement. Les résultats montrent que les composés de la présente invention augmentent l'éveil (diminution des ondes delta) cortical chez le rat.

A titre d'exemple, le composé de l'Exemple 1 administré à la dose de 3 mg/kg IP, induit une diminution significative de la puissance des ondes lentes delta pendant 120 minutes, signe d'activation cortical et d'éveil.

### EXEMPLE C : Interaction au barbital chez le rat Wistar

L'objectif de ce test est de déterminer les propriétés anti-sédatives, éveillantes et/ou anti-hypnotiques des composés de la présente invention. Les rats sont placés dans des cages individuelles et reçoivent une injection de barbital (170 mg/kg IP). La durée de sommeil est ensuite mesurée pendant 4 heures après l'injection de barbital. Elle est déterminée par la perte du réflexe de retournement. Les composés de l'invention ou leurs véhicules sont administrés par voie orale 30 minutes avant l'administration de barbital. Les résultats indiquent que les composés de la présente invention possèdent de puissantes activités anti-sédatives, anti-hypnotiques et/ou éveillantes.

Par exemple, à la dose de 10 mg/kg PO, le composé de l'Exemple 1 diminue la durée d'endormissement induite au barbital de -81 %.

### EXEMPLE D : Reconnaissance d'objet chez le Rat Sprague-Dawley

Le test de la reconnaissance d'objet chez le rat Sprague-Dawley (Behav. Brain Res., 1988, 31, 47-59) est basé sur l'activité exploratoire spontanée de l'animal et possède les caractéristiques de la mémoire épisodique chez l'homme. Sensible au vieillissement (Eur. J. Pharmacol., 1997, 325, 173-180), ainsi qu'aux dysfonctionnements cholinergiques (Pharm. Biochem. Behav., 1996, 53(2), 277-283), ce test de mémoire est fondé sur l'exploration différentielle de 2 objets de forme assez similaire, l'un familier, l'autre nouveau. Préalablement au test, les animaux sont habitués à l'environnement (enceinte sans objet). Au cours d'une 1^{ère} session, les rats sont placés (3 minutes) dans l'enceinte où se trouvent 2 objets identiques. La durée d'exploration de chaque objet est mesurée. Au cours de la 2^{ème} session (3 minutes), 24 heures plus tard, 1 des 2 objets est remplacé par un nouvel objet. La durée d'exploration de chaque objet est mesurée. Le critère de jugement est la différence Delta, exprimée en seconde, des temps d'exploration de l'objet nouveau et de l'objet familier au cours de la 2^{ème} session. Les animaux témoins, traités préalablement au véhicule par voie orale 60 minutes avant chaque session, explorent de façon identique l'objet familier et l'objet nouveau, ce qui signe l'oubli de l'objet déjà présenté. Les animaux traités par un composé facilitateur mnémocognitif, explore de façon préférentielle l'objet nouveau, ce qui signe le souvenir de l'objet déjà présenté.

Par exemple, les résultats obtenus avec l'Exemple 1 de la présente invention montrent une différence Delta de l'ordre de 8 secondes à la dose de 3 mg/kg PO, ce qui indique que les composés de l'invention améliorent la mémorisation de façon importante, et à faible dose.

### EXEMPLE E : Reconnaissance sociale chez le Rat Wistar

Initialement décrit en 1982 (J. Comp. Physiol., 1982, 96, 1000-1006), le test de la reconnaissance sociale a été ensuite proposé par différents auteurs (Psychopharmacology, 1987, 91, 363-368) ; Psychopharmacology, 1989, 97, 262-268) pour l'étude des effets mnémocognitifs de nouveaux composés. Fondé sur l'expression naturelle de la mémoire olfactive du rat et sur son oubli naturel, ce test permet d'apprécier la mémorisation, par la reconnaissance d'un jeune congénère, par un rat adulte. Un jeune rat (21 jours), pris au hasard, est placé dans la cage de stabulation d'un rat adulte pendant 5 minutes. Par l'intermédiaire d'un dispositif vidéo, l'expérimentateur observe le comportement de reconnaissance sociale du rat adulte et en mesure la durée globale. Puis le jeune rat est ôté de la cage du rat adulte et est placé dans une cage individuelle, jusqu'à la seconde présentation. Le rat adulte reçoit alors le produit à tester par voie intrapéritonéale et, 2 heures plus tard, est remis en présence (5 minutes) du jeune rat. Le comportement de reconnaissance sociale est alors à nouveau observé et la durée en est mesurée. Le critère de jugement est la différence (T2-T1), exprimée en secondes, des temps de « reconnaissance » des 2 rencontres.

Les résultats obtenus avec l'Exemple 1 montrent une différence (T2-T1) de 24 secondes et de 36 secondes pour des doses de 1 et 3 mg/kg IP, respectivement. Ceci montre que les composés de l'invention augmentent la mémorisation de façon très importante, et à faible dose.

### EXEMPLE F : Test de la suspension caudale chez la souris NMRI

Le test de suspension caudale chez la souris (Porsolt et al., Arch. Int. Pharmacodyn., 1987, 288, 11) permet la détection de propriétés psychopharmacologiques de composés. Des souris NMRI sont suspendues par la queue, à l'aide d'un morceau de scotch, à un crochet pendant 6 minutes : le temps d'immobilité est mesurée automatiquement à l'aide de capteurs de mouvements. Les animaux sont traités par voie intrapéritonéale 24 heures et 30 minutes avant leur suspension par les composés de l'invention ou leurs véhicules.

Les résultats montrent que l'Exemple 1, administré à la dose de 10 mg/kg IP, induit une augmentation du temps d'immobilité de 119 %. Ce résultat indique que les composés de la présente invention possèdent des propriétés anxiolytiques

### EXEMPLE G : Microdialyse intracérébrale dans le striatum de rat vigile

La microdialyse intracérébrale chez le rat vigile permet d'évaluer l'influence d'un composé sur la libération de neurotransmetteurs, comme la dopamine, dans l'espace extracellulaire de petites structures cérébrales, notamment le striatum.
Cette technique est réalisée selon deux étapes : une étape de chirurgie (implantation stéréotaxique d'une canule-guide, sur animal anesthésié) et une étape de microdialyse sur animal vigile (recueil d'échantillons de liquide cérébral extracellulaire).
Des rats mâles de souche Wistar (280-320 g) sont anesthésiés et sont placés dan un appareil de stéréotaxie. Une canule-guide est implantée dans le striatum des animaux selon les coordonnées stéréotaxiques suivantes : antéro-postériorité -1 mm, latéralité +2.8 mm, profondeur -3 mm par rapport au bregma, d'après l'atlas de Paxinos et Watson (1996). Une semaine après la chirurgie, une sonde de microdialyse (CMA11, longueur 4 mm, Phymep) est introduite dans la canule-guide. Les animaux sont placés dans des cages d'expérimentation et l'entrée de la sonde est connectée à une pompe qui perfuse en continu du liquide céphalo-rachidien artificiel (débit 1 µl/min). Après une période de stabilisation de 2 heures, la collecte des microdialysats débute. Des échantillons sont recueillis dans les conditions basales (4 échantillons) puis après administration intrapéritonéale du composé (6 échantillons post-traitement).
Dans chaque microdialysat, les taux extracellulaires de dopamine sont évalués à l'aide d'une technique de chromatographie liquide couplée à une détection électrochimique. Les valeurs sont exprimées en moyenne ± SEM par rapport aux valeurs basales (référence 100 %).
Les résultats montrent que l'Exemple 1 de la présente invention est capable d'augmenter significativement les concentrations cérébrales endogènes de dopamine de +123% (par rapport aux valeurs basales) à la dose de 10 mg/kg i.p.

### EXEMPLE H : Compositions pharmaceutiques

Formule de préparation pour 1000 comprimés dosés à 100 mg :

| | |
|---|---|
| Composé de l'Exemple 1 | 100 g |
| Hydroxypropylcellulose | 20 g |
| Polyvinylpyrrolidone | 20 g |
| Amidon de blé | 150 g |
| Lactose | 900 g |
| Stéarate de magnésium | 30 g |

## Revendications

1. Composés de formule (I) : 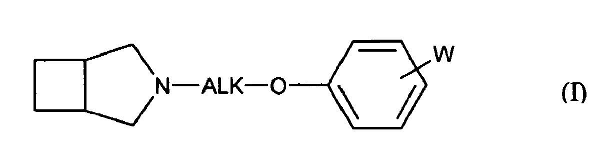 dans laquelle :
◆ ALK représente une chaîne alkylène,
◆ W représente un groupement 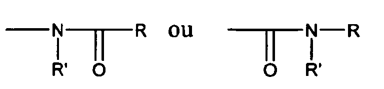 où R et R' représentent, indépendamment l'un de l'autre, un atome d'hydrogène ou un groupement alkyle (C₁-C₆) linéaire ou ramifié éventuellement substitué par un ou plusieurs groupements choisis parmi halogène, hydroxy et alkoxy,
étant entendu que :
- le terme alkylène désigne un radical bivalent, linéaire ou ramifié, contenant de 2 à 6 atomes de carbone,
- le terme alkoxy désigne un groupement alkyle-oxy dont la chaîne alkyle, linéaire ou ramifiée, contient de 1 à 6 atomes de carbone,
leurs énantiomères et diastéréoisomères, ainsi que leurs sels d'addition à un acide ou à une base pharmaceutiquement acceptable.

2. Composés de formule (I) selon la revendication 1 pour lesquels le groupement W est situé en position para.

3. Composés de formule (I) selon la revendication 1 pour lesquels ALK représente un radical bivalent linéaire contenant de 2 à 4 atomes de carbone, leurs énantiomères et diastéréoisomères ainsi que leurs sels d'addition à un acide ou à une base pharmaceutiquement acceptables.

4. Composés de formule (I) selon la revendication 1 pour lesquels ALK représente un groupement propylène, leurs énantiomères et diastéréoisomères ainsi que leurs sels d'addition à un acide ou à une base pharmaceutiquement acceptables.

5. Composés de formule (I) selon la revendication 1 pour lesquels W représente un groupement 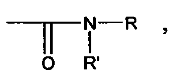 leurs énantiomères et diastéréoisomères ainsi que leurs sels d'addition à un acide ou à une base pharmaceutiquement acceptables.

6. Composés de formule (I) selon la revendication 1 pour lesquels W représente un groupement 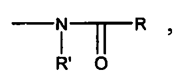 leurs énantiomères et diastéréoisomères ainsi que leurs sels d'addition à un acide ou à une base pharmaceutiquement acceptables.

7. Composés de formule (I) selon la revendication 1 pour lesquels R et R' représentent, indépendamment l'un de l'autre, un atome d'hydrogène, un groupement méthyle ou un groupement éthyle, ces groupements étant éventuellement substitués par un méthoxy, leurs énantiomères et diastéréoisomères ainsi que leurs sels d'addition à un acide ou à une base pharmaceutiquement acceptables.

8. Composés de formule (I) selon la revendication 1 pour lesquels W représente un groupement -CO-NH₂, -CO-NH-CH₃, -CO-N(CH₃)₂, -CO-N(CH₂CH₃)₂, NH-CO-CH₃, 3-N(CH₃)-CO-CH₃ ou -NH-CO-CH₂-OCH₃, leurs énantiomères et diastéréoisomères ainsi que leurs sels d'addition à un acide ou à une base pharmaceutiquement acceptables.

9. Composés de formule (I) selon la revendication 1 qui sont :
- le 4-{3-[(1*R*,5*S*)-3-azabicyclo[3.2.0]hept-3-yl]propoxy}benzamide,
- le *N*-(4-{3-[(1*R*,5*S*)-3-azabicyclo[3.2.0]hept-3-yl]propoxy}phényl)acétamide,
- le 4-{3-[(1*R*,5*S*)-3-azabicyclo[3.2.0]hept-3-yl]propoxy}-*N,N*-diméthylbenzamide,
- le *N*-(4-{3-[(1*R*,5*S*)-3-azabicyclo[3.2.0]hept-3-yl]propoxy)phényl)-*N*-méthyl acétamide,
et leurs sels d'addition à un acide ou à une base pharmaceutiquement acceptables.

10. Composés de formule (I) selon la revendication 1 qui sont :
- le chlorhydrate de 4-{3-[(1*R*,5*S*)-3-azabicyclo[3.2.0]hept-3-yl]propoxy}benzamide,
- l'oxalate de 4-{3-[(1*R*,5*S*)-3-azabicyclo[3.2.0]hept-3-yl]propoxy}benzamide,
- le citrate de 4-{3-[(1*R*,5*S*)-3-azabicyclo[3.2.0]hept-3-yl]propoxy}benzamide.

11. Procédé de préparation des composés de formule (I) selon la revendication 1 **caractérisé en ce que** l'on utilise comme produit de départ le composé de formule (II) : 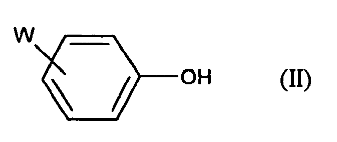 dans laquelle W est tel que défini dans la revendication 1,
composé de formule (II) sur lequel on condense en milieu basique le composé de formule (III) :
Br-ALK-Cl (III)
dans laquelle ALK est tel que défini dans la formule (I),
pour obtenir le composé de formule (IV) : 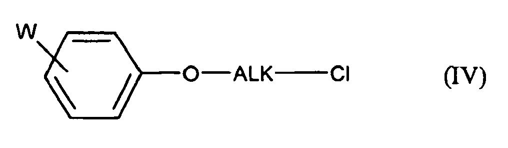 dans laquelle W et ALK sont tels que définis précédemment,
sur lequel on condense le composé de formule (V) : 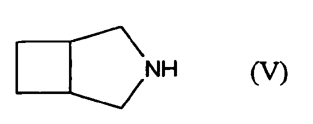 pour conduire au composé de formule (I) tel que défini précédemment : 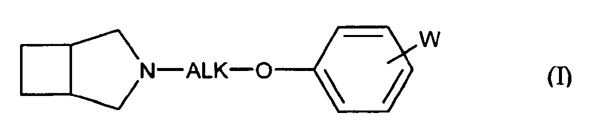 qui peut être purifié selon une technique classique de séparation, que l'on transforme, si on le souhaite, en ses sels d'addition à un acide ou à une base pharmaceutiquement acceptable et dont on sépare éventuellement les isomères optiques selon une technique classique de séparation.

12. Composés de formule (VI) suivante : 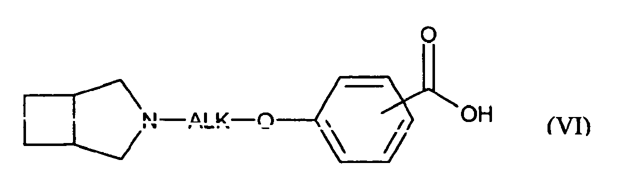 dans lesquels le groupement ALK est tel que défini dans la revendication 1,
utiles en tant qu'intermédiaires de synthèse des composés de formule (I/a), cas particuliers des composés de formule (I) selon la revendication 1 pour lesquels W représente un groupement -CONRR', étant donné que W, R et R' sont tels que définis dans la revendication 1.

13. Composés de formule (VII) suivante : 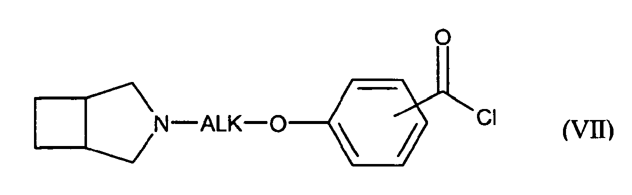 dans lesquels le groupement ALK est tel que défini dans la revendication 1,
utiles en tant qu'intermédiaires de synthèse des composés de formule (I/a), cas particuliers des composés de formule (I) selon la revendication 1 pour lesquels W représente un groupement -CONRR', étant donné que W, R et R' sont tels que définis dans la revendication 1.

14. Composés de formule (VIII) suivante : 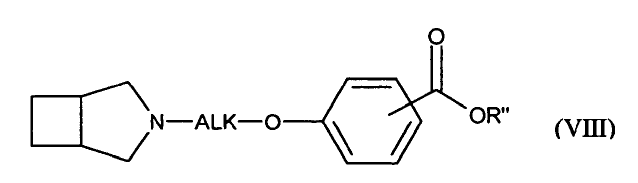 dans lesquels le groupement ALK est tel que défini dans la revendication 1 et R" est un groupe alkyle (C₁-C₆) linéaire ou ramifié ou un groupement benzyle,
utiles en tant qu'intermédiaires de synthèse des composés de formule (I/a), cas particuliers des composés de formule (I) selon la revendication 1 pour lesquels W représente un groupement -CONRR', étant donné que W, R et R' sont tels que définis dans la revendication 1.

15. Composés de formule (IX) suivante : 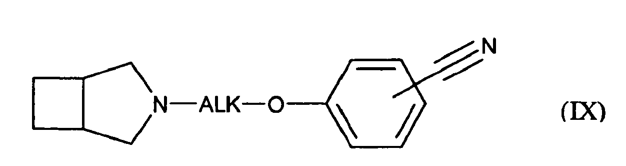 dans lesquels le groupement ALK est tel que défini dans la revendication 1,
utiles en tant qu'intermédiaires de synthèse des composés de formule (I/a), cas particuliers des composés de formule (I) selon la revendication 1 pour lesquels W représente un groupement -CONRR', étant donné que W, R et R' sont tels que définis dans la revendication 1.

16. Compositions pharmaceutiques contenant comme principe actif un composé de formule (I) selon l'une quelconque des revendications 1 à 10, ou un de ses sels d'addition à un acide ou à une base pharmaceutiquement acceptables, en combinaison avec un ou plusieurs excipients pharmaceutiquement acceptables.

17. Compositions pharmaceutiques selon la revendication 16 pour son utilisation dans le traitement des troubles cognitifs et psychocomportementaux liés au vieillissement cérébral, aux maladies neurodégénératives ou aux traumatismes crâniens.

18. Compositions pharmaceutiques selon la revendication 17 pour son utilisation dans le traitement des troubles cognitifs et psychocomportementaux associés à la maladie d'Alzheimer, la maladie de Parkinson, la maladie de Pick, les démences à corps de Lewy, les démences frontales et sous-corticales, les démences frontotemporales, les démences vasculaires, la maladie de Huntington, et la sclérose en plaques.

19. Compositions pharmaceutiques selon la revendication 17 pour son utilisation dans le traitement des troubles psychocomportementaux tels que les troubles du sommeil, l'apathie et les états anxio-dépressifs.

20. Compositions pharmaceutiques selon la revendication 19 pour son utilisation dans le traitement des troubles du sommeil associés à la maladie d'Alzheimer et à la maladie de Parkinson.

21. Compositions pharmaceutiques selon la revendication 16 pour son utilisation dans le traitement des troubles moteurs associés à la maladie de Parkinson.

22. Compositions pharmaceutiques selon la revendication 16 pour son utilisation dans le traitement des troubles de l'humeur, des états anxio-dépressifs, du syndrome de Tourette, de la schizophrénie et des troubles cognitifs qui lui sont associés, de la douleur, ainsi que dans le traitement des troubles du sommeil, du rythme veille-sommeil et du syndrome d'hyperactivité avec déficits attentionnels.

23. Compositions pharmaceutiques selon la revendication 22 pour son utilisation dans le traitement des troubles du sommeil tels que la narcolepsie, les hypersomnies survenant lors du syndrome d'apnée obstructive du sommeil ou du syndrome d'hyperactivité avec déficits attentionnels, ainsi que les somnolences diurnes.

24. Association d'un composé de formule (I) selon l'une quelconque des revendications 1 à 10 avec la L-dopa.

25. Association selon la revendication 24 pour son utilisation dans le traitement des troubles cognitifs et moteurs de la maladie de Parkinson.

## Claims

1. Compounds of formula (I): 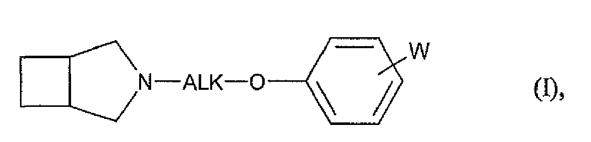 wherein:
◆ ALK represents an alkylene chain,
◆ W represents a group 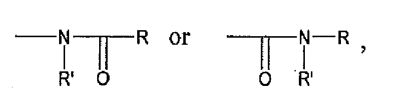 in which R and R', each independently of the other, represent a hydrogen atom or a linear or branched (C₁-C₆)alkyl group optionally substituted by one or more groups selected from halogen, hydroxy and alkoxy,
it being understood that:
- the term "alkylene" denotes a linear or branched divalent radical containing from 2 to 6 carbon atoms,
- the term "alkoxy" denotes an alkyl-oxy group in which the alkyl chain, which is linear or branched, contains from 1 to 6 carbon atoms,
their enantiomers and diastereoisomers, and also addition salts thereof with a pharmaceutically acceptable acid or base.

2. Compounds of formula (I) according to claim 1, wherein the W group is located in the para position.

3. Compounds of formula (I) according to claim 1, wherein ALK represents a linear divalent radical containing from 2 to 4 carbon atoms, their enantiomers and diastereoisomers, and also addition salts thereof with a pharmaceutically acceptable acid or base.

4. Compounds of formula (I) according to claim 1, wherein ALK represents a propylene group, their enantiomers and diastereoisomers, and also addition salts thereof with a pharmaceutically acceptable acid or base.

5. Compounds of formula (I) according to claim 1, wherein W represents a group 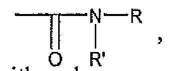 their enantiomers and diastereoisomers, and also addition salts thereof with a pharmaceutically acceptable acid or base.

6. Compounds of formula (I) according to claim 1, wherein W represents a group 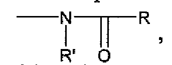 their enantiomers and diastereoisomers, and also addition salts thereof with a pharmaceutically acceptable acid or base.

7. Compounds of formula (I) according to claim 1, wherein R and R', each independently of the other, represent a hydrogen atom, a methyl group or an ethyl group, those groups optionally being substituted by a methoxy group, their enantiomers and diastereoisomers, and also addition salts thereof with a pharmaceutically acceptable acid or base.

8. Compounds of formule (I) according to claim 1, wherein W represents a group -CO-NH₂, -CO-NH-CH₃, -CO-N(CH₃)₂, -CO-N(CH₂CH₃)₂, -NH-CO-CH₃, -N(CH₃)-CO-CH₃ or NH-CO-CH₂-OCH₃, their enantiomers and diastereoisomers, and also addition salts thereof with a pharmaceutically acceptable acid or base.

9. Compounds of formula (I) according to claim 1, which are:
- 4-{3-[(1*R*,5*S*)-3-azabicyclo[3.2.0]hept-3-yl]propoxy}benzamide,
- *N*-(4-{3-[(1*R*,5*S*)-3-azabicyclo[3.2.0]hept-3-yl]propoxy}phenyl)acetamide,
- 4-{3-[(1*R*,5*S*)-3-azabicyclo[3.2.0]hept-3-yl]propoxy}-*N,N-*dimethylbenzamide,
- *N*-(4-{3-[(1*R*,5*S*)-3-azabicyclo[3.2.0]hept-3-yl]propoxy}phenyl)-*N*-methylacetamide,
and addition salts thereof with a pharmaceutically acceptable acid or base.

10. Compounds of formula (I) according to claim 1, which are:
- 4-{3-[(1*R*,5*S*)-3-azabicyclo[3.2.0]hept-3-yl]propoxy}benzamide hydrochloride,
- 4-{3-[(1*R*,5*S*)-3-azabicyclo[3.2.0]hept-3-yl]propoxy}benzamide oxalate,
- 4-{3-[(1*R*,5*S*)-3-azabicyclo[3.2.0]hept-3-yl]propoxy]benzamide citrate.

11. Process for the preparation of compounds of formula (I) according to claim 1, **characterised in that** there is used as starting material the compound of formula (II): 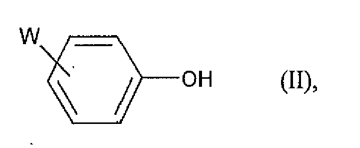 wherein W is as defined in claim 1,
with which compound of formula (II) there is condensed, in a basic medium, the compound of formula (III):
Br—ALK———Cl (III),
wherein ALK is as defined for formula (I),
to obtain the compound of formula (IV): 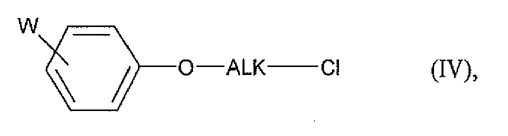 wherein W and ALK are as defined hereinbefore,
with which there is condensed the compound of formula (V): 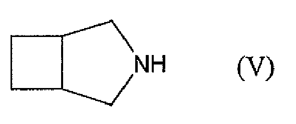 to yield the compound of formula (I) as defined hereinbefore: 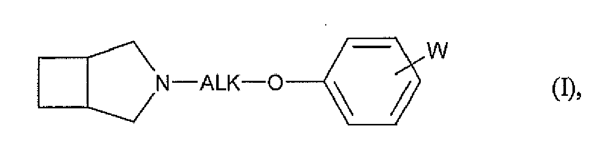 which may be purified according to a conventional separation technique, is converted, if desired, into its addition salts with a pharmaceutically acceptable acid or base and is separated, where appropriate, into its optical isomers according to a conventional separation technique.

12. Compounds of the following formula (VI): 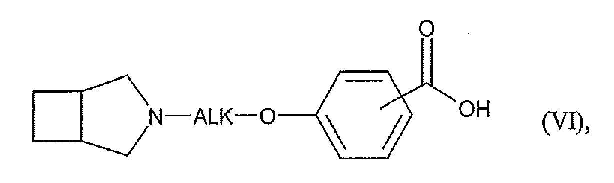 wherein the ALK group is as defined in claim 1,
for use as synthesis intermediates for compounds of formula (I/a), particular cases of compounds of formula (I) according to claim 1, wherein W represents a group -CONRR', it being understood that W, R and R' are as defined in claim 1.

13. Compounds of the following formula (VII): 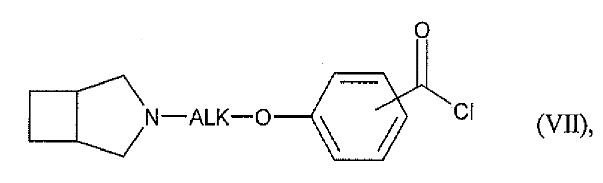 wherein the ALK group is as defined in claim 1,
for use as synthesis intermediates for compounds of formula (I/a), particular cases of compounds of formula (I) according to claim 1, wherein W represents a group -CONRR', it being understood that W, R and R' are as defined in claim 1.

14. Compounds of the following formula (VIII): 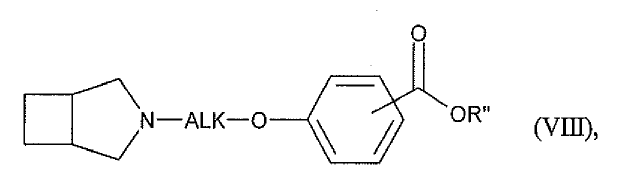 wherein the ALK group is as defined in claim 1 and R" is a linear or branched (C₁-C₆)alkyl group or a benzyl group,
for use as synthesis intermediates for compounds of formula (I/a), particular cases of compounds of formula (I) according to claim 1, wherein W represents a group -CONRR', it being understood that W, R and R' are as defined in claim 1.

15. Compounds of the following formula (IX): 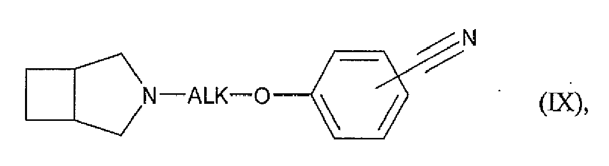 wherein the ALK group is as defined in claim 1,
for use as synthesis intermediates for compounds of formula (I/a), particular cases of compounds of formula (I) according to claim 1, wherein W represents a group -CONRR', it being understood that W, R and R' are as defined in claim 1,

16. Pharmaceutical compositions comprising as active ingredient a compound of formula (I) according to any one of claims 1 to 10, or an addition salt thereof with a pharmaceutically acceptable acid or base, in combination with one or more pharmaceutically acceptable excipients.

17. Pharmaceutical compositions according to claim 16 for use in the treatment of cognitive and psycho-behavioural disorders associated with cerebral ageing, with neurodegenerative diseases or with cranial traumas.

18. Pharmaceutical compositions according to claim 17 for use in the treatment of cognitive and psycho-behavioural disorders associated with Alzheimer's disease, Parkinson's disease, Pick's disease, Lewy body dementias, frontal and subcortical dementias, frontotemporal dementias, vascular dementias, Huntington's disease and multiple sclerosis.

19. Pharmaceutical compositions according to claim 17 for use in the treatment of psycho-behavioural disorders such as sleep disorders, apathy and anxio-depressive states.

20. Pharmaceutical compositions according to claim 19 for use in the treatment of sleep disorders associated with Alzheimer's disease and with Parkinson's disease.

21. Pharmaceutical compositions according to claim 16 for use in the treatment of motor disorders associated with Parkinson's disease.

22. Pharmaceutical compositions according to claim 16 for use in the treatment of mood disorders, of anxio-depressive states, of Tourette's syndrome, of schizophrenia and of cognitive disorders associated therewith, and of pain, and also in the treatment of sleep disorders, of sleep-waking rhythm disorders and of attention-deficit hyperactivity syndrome.

23. Pharmaceutical compositions according to claim 22 for use in the treatment of sleep disorders such as narcolepsy, hypersomnia occurring in obstructive sleep apnoea syndrome or in attention-deficit hyperactivity syndrome, and also diurnal somnolence.

24. Association of a compound of formula (I) according to any one of claims 1 to 10 with L-dopa.

25. Association according to claim 24 for use in the treatment of cognitive and motor disorders of Parkinson's disease.

## Patentansprüche

1. Verbindungen der Formel (I): 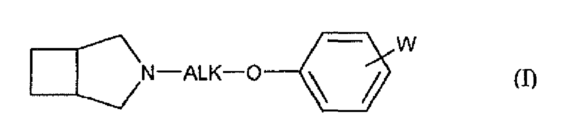 in der:
◆ ALK eine Alkylenkette bedeutet,
◆ W eine Gruppe 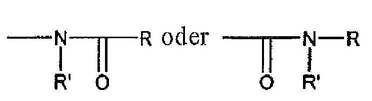 bedeutet,
worin R und R' unabhängig voneinander ein Wasserstoffatom oder eine geradkettige oder verzweigte, gegebenenfalls durch eine oder mehrere Gruppen ausgewählt aus Halogen, Hydroxy und Alkoxy substituierte (C₁-C₆)-Alkylgruppe bedeuten,
mit der Maßgabe, dass:
- der Begriff Alkylen für einen zweiwertigen, geradkettigen oder verzweigten Rest steht, der 2 bis 6 Kohlenstoffatome enthält,
- der Begriff Alkoxy für eine Alkyloxygruppe steht, deren geradkettige oder verzweigte Alkylkette 1 bis 6 Kohlenstoffatome enthält,
deren Enantiomere und Diastereoisomere sowie deren Additionssalze mit einer pharmazeutisch annehmbaren Säure oder Base.

2. Verbindungen der Formel (I) nach Anspruch 1, worin die Gruppe W in der para-Stellung steht.

3. Verbindungen der Formel (I) nach Anspruch 1, worin ALK einen zweiwertigen, geradkettigen Rest bedeutet, der 2 bis 4 Kohlenstoffatome enthält, deren Enantiomere und Diastereoisomere sowie deren Additionssalze mit einer pharmazeutisch annehmbaren Säure oder Base.

4. Verbindungen der Formel (I) nach Anspruch 1, worin ALK eine Propylengruppe bedeutet, deren Enantiomere und Diastereoisomere sowie deren Additionssalze mit einer pharmazeutisch annehmbaren Säure oder Base.

5. Verbindungen der Formel (I) nach Anspruch 1, worin W eine Gruppe 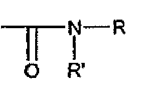 bedeutet, deren Enantiomere und Diastereoisomere sowie deren Additionssalze mit einer pharmazeutisch annehmbaren Säure oder Base.

6. Verbindungen der Formel (I) nach Anspruch 1, worin W eine Gruppe 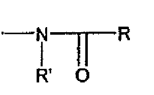 bedeutet, deren Enantiomere und Diastereoisomere sowie deren Additionssalze mit einer pharmazeutisch annehmbaren Säure oder Base.

7. Verbindungen der Formel (I) nach Anspruch 1, worin R und R' unabhängig voneinander ein Wasserstoffatom, eine Methylgruppe oder eine Ethylgruppe bedeuten, wobei diese Gruppen gegebenenfalls durch eine Methoxygruppe substituiert sind, deren Enantiomere und Diastereoisomere sowie deren Additionssalze mit einer pharmazeutisch annehmbaren Säure oder Base.

8. Verbindungen der Formel (I) nach Anspruch 1, worin W eine Gruppe -CO-NH₂, -CO-NH-CH₃, -CO-N(CH₃)₂, -CO-N(CH₂CH₃)₂, -NH-CO-CH₃, -N(CH₃)-CO-CH₃ oder -NH-CO-CH₂-OCH₃ bedeutet, deren Enantiomere und Diastereoisomere sowie deren Additionssalze mit einer pharmazeutisch annehmbaren Säure oder Base.

9. Verbindungen der Formel (I) nach Anspruch 1, nämlich:
- 4-{3-[(1*R*,5*S*)-3-Azabicyclo[3.2.0]hept-3-yl]-propoxy}-benzamid,
- *N*-(4-{3-[(1*R*,5*S*)-3-Azabicyclo[3.2.0]hept-3-yl]-propoxy}-phenyl)-acetamid,
- 4-{3-[(1*R*,5*S*)-3-Azabicyclo[3.2.0]hept-3-yl]-propoxyl-*N,N*-dimethylbenzamid,
- *N*-(4-{3-[(1*R*,5*S*)-3-Azabicyclo[3.2.0]hept-3-yl]-propoxy}-phenyl)-*N*-methyl-acetamid
und deren Additionssalze mit einer pharmazeutisch annehmbaren Säure oder Base.

10. Verbindungen der Formel (I) nach Anspruch 1, nämlich:
- das Hydrochlorid von 4-{3-[(1*R*,5*S*)-3-Azabicyclo[3.2.0]hept-3-yl]-propoxy}-benzamid,
- das Oxalat von 4-{3-[(1*R*,5*S*)-3-Azabicyclo[3.2.0]hept-3-yl]-propoxy}-benzamid,
- das Citrat von 4-{3-[(1*R*,5*S*)-3-Azabicyclo[3.2.0]hept-3-yl]-propoxy}-benzamid.

11. Verfahren zur Herstellung der Verbindungen der Formel (I) nach Anspruch 1, **dadurch gekennzeichnet, dass** man als Ausgangsprodukt die Verbindung der Formel (II) verwendet: 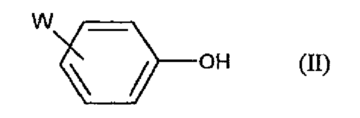 in der W die in Anspruch 1 angegebenen Bedeutungen besitzt,
welche Verbindung der Formel (II) man in basischem Medium mit der Verbindung der Formel (III) kondensiert:
Br - ALK - Cl **(III)**
in der ALK die bezüglich der Formel (I) angegebenen Bedeutungen besitzt,
zur Bildung der Verbindung der Formel (IV): 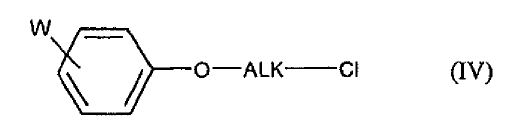 in der W und ALK die oben angegebenen Bedeutungen besitzen,
welche man mit der Verbindung der Formel (V) kondensiert: 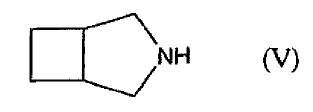 zur Bildung der Verbindung der Formel (I), wie sie oben definiert worden ist: 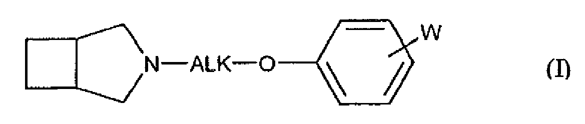 welche mit Hilfe einer klassischen Trennungsmethode gereinigt werden kann, welche man gewünschtenfalls in ihre Additionssalze mit einer pharmazeutisch annehmbaren Säure oder Base überführt oder deren optische Isomeren man gegebenenfalls mit Hilfe einer klassischen Trennungsmethode trennt.

12. Verbindungen der folgenden Formel (VI): 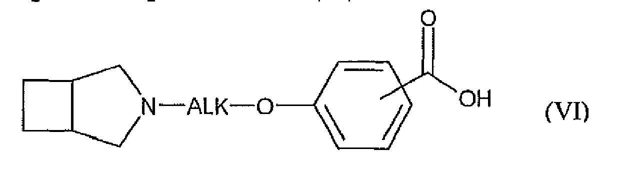 in der die Gruppe ALK die in Anspruch 1 angegebenen Bedeutungen besitzt,
nützlich als Zwischenprodukte bei der Synthese der Verbindungen der Formel (I/a), einem Sonderfall der Verbindungen der Formel (I) nach Anspruch 1, worin W eine Gruppe -CONRR' bedeutet, mit der Maßgabe, dass W, R und R' die in Anspruch 1 angegebenen Bedeutungen besitzen.

13. Verbindungen der folgenden Formel (VII): 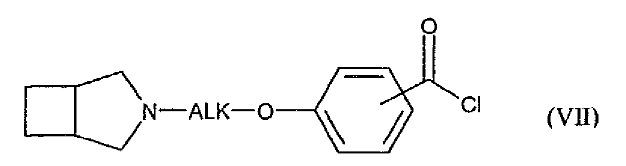 in der die Gruppe ALK die in Anspruch 1 angegebenen Bedeutungen besitzt,
nützlich als Zwischenprodukte bei der Synthese der Verbindungen der Formel (I/a), einem Sonderfall der Verbindungen der Formel (I) nach Anspruch 1, worin W eine Gruppe -CONRR' bedeutet, mit der Maßgabe, dass W, R und R' die in Anspruch 1 angegebenen Bedeutungen besitzen.

14. Verbindungen der folgenden Formel (VIII): 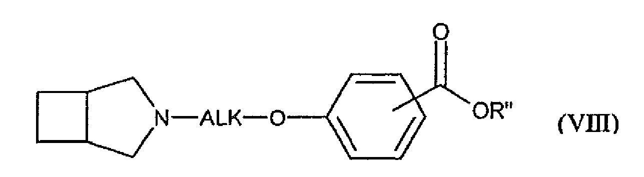 in der die Gruppe ALK die in Anspruch 1 angegebenen Bedeutungen besitzt und R" eine geradkettige oder verzweigte (C₁-C₆)-Alkylgruppe oder eine Benzylgruppe bedeutet,
nützlich als Zwischenprodukte bei der Synthese der Verbindungen der Formel (I/a), einem Sonderfall der Verbindungen der Formel (I) nach Anspruch 1, worin W eine Gruppe -CONRR' bedeutet, mit der Maßgabe, dass W, R und R' die in Anspruch 1 angegebenen Bedeutungen besitzen.

15. Verbindungen der folgenden Formel (IX): 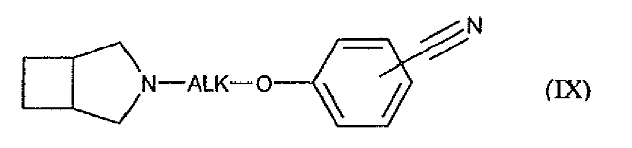 in der die Gruppe ALK die in Anspruch 1 angegebenen Bedeutungen besitzt,
nützlich als Zwischenprodukte bei der Synthese der Verbindungen der Formel (I/a), einem Sonderfall der Verbindungen der Formel (I) nach Anspruch 1, worin W eine Gruppe -CONRR' bedeutet, mit der Maßgabe, dass W, R und R' die in Anspruch 1 angegebenen Bedeutungen besitzen.

16. Pharmazeutische Zubereitungen enthaltend als Wirkstoff eine Verbindung der Formel (I) nach einem der Ansprüche 1 bis 10 oder eines ihrer Additionssalze mit einer pharmazeutisch annehmbaren Säure oder Base in Kombination mit einem oder mehreren pharmazeutisch annehmbaren Hilfsstoffen.

17. Pharmazeutische Zubereitungen nach Anspruch 16 zur Verwendung bei der Behandlung von kognitiven oder psychoverhaltensmäßigen Störungen, die verknüpft sind mit dem Altern des Gehirns, mit neurodegenerativen Erkrankungen oder mit Schädeltraumata.

18. Pharmazeutische Zubereitungen nach Anspruch 17 zur Verwendung bei der Behandlung von kognitiven und psychoverhaltensmäßigen Störungen, die verknüpft sind mit der Alzheimerschen Krankheit, der Parkinsonschen Krankheit, der Pickschen Krankheit, Lewy-Körperchen-Demenz, frontalen und subkortikalen Demenzen, frontotemporalen Demenzen, vaskulären Demenzen, der Huntington-Krankheit und der Multiplen Sklerose.

19. Pharmazeutische Zubereitungen nach Anspruch 17 zur Verwendung bei der Behandlung von psychoverhaltensmäßigen Störungen, wie Schlafstörungen, Apathie und depressiven Angstzuständen.

20. Pharmazeutische Zubereitungen nach Anspruch 19 zur Verwendung bei der Behandlung von Schlafstörungen, die mit der Alzheimerschen Krankheit und der Parkinsonschen Krankheit verknüpft sind.

21. Pharmazeutische Zubereitungen nach Anspruch 16 zur Verwendung bei der Behandlung von mit der Parkinsonschen Krankheit verknüpften motorischen Störungen.

22. Pharmazeutische Zubereitungen nach Anspruch 16 zur Verwendung bei der Behandlung von Gemütsstörungen, depressiven Angstzuständen, dem Tourette-Syndrom, der Schizophrenie und damit verknüpften kognitiven Störungen, Schmerzen sowie bei der Behandlung von Störungen des Schlafes, des Wach-Schlaf-Rhythmus oder des Hyperaktivitätssyndroms mit Aufmerksamkeitsdefiziten.

23. Pharmazeutische Zubereitungen nach Anspruch 22 zur Verwendung bei der Behandlung von Schlafstörungen, wie Narkolepsie, Hypersomnien, die beim obstruktiven Schlafapnoesyndrom oder dem Hyperaktivitätssyndrom mit Aufinerksamkeitsdefiziten auftreten, sowie der Tagesschlafsucht.

24. Kombination einer Verbindung der Formel (I) nach einem der Ansprüche 1 bis 10 mit L-Dopa.

25. Kombination nach Anspruch 24 zur Verwendung bei der Behandlung von kognitiven und motorischen Störungen der Parkinsonschen Krankheit.
